# EUROPÄISCHE PATENTANMELDUNG

(11) **EP 4 505 963 A1**
(43) Veröffentlichungstag der Anmeldung: **12.02.2025**
(21) Anmeldenummer: 23190298.2
(22) Anmeldetag: 08.08.2023
(51) Int. Cl.: A61B 34/30, A61B 90/11, A61B 5/055, A61B 90/00

(54) **MAGNETRESONANZTOMOGRAPH**

(71) Anmelder: Siemens Healthineers AG, 91301 Forchheim (DE)
(72) Erfinder: Dr. Hengerer, Arne, 91096 Möhrendorf (DE)
(74) Vertreter: Siemens Healthineers Patent Attorneys

(57) **Zusammenfassung**

Die Erfindung betrifft einen Magnetresonanztomographen (10) umfassend einen Patiententisch (15), einen Patientenaufnahmebereich (12), eine Vorschubeinheit (42), die dazu ausgebildet ist, ein medizintechnisches Instrument (41) zu führen und ein Schienensystem (43) zum Führen der Vorschubeinheit (42). Dabei verläuft das Schienensystem (43) innenumfänglich entlang einer Innenseite (37) des Patientenaufnahmebereiches (12). Dabei ist der Patiententisch (15) dazu ausgebildet, einen Patienten (13) in dem Patientenaufnahmebereich (12) zu positionieren Dabei ist der Patiententisch (15) derart bewegbar ausgebildet, dass das medizintechnische Instrument (41) anhand einer Bewegung des Patiententisches (15) in dem Patienten (13) entlang einer Trajektorie geführt werden kann.

## Beschreibung

Die vorliegende Erfindung betrifft einen Magnetresonanztomographen, ein Verfahren zum Planen einer Bewegung eines medizintechnischen Instrumentes in einem Patientenaufnahmebereich eines Magnetresonanztomographen, ein Planungssystem, welches ausgebildet ist, das Verfahren auszuführen, ein Computerprogrammprodukt, sowie ein computerlesbares Speichermedium.

Es ist bekannt, eine Intervention an einem Patienten mit einer mit einem Magnetresonanztomographen ausgeführten medizinischen Bildgebung zu überwachen. Dies wird auch als bildgestützte Intervention bezeichnet. Dafür ist der Patient in einem Patientenaufnahmebereich in dem Magnetresonanztomographen positioniert. Der Patientenaufnahmebereich wird typischerweise durch eine Umhausung des Magnetresonanztomographen gebildet und ist typischerweise zylinderförmig. Die Intervention wird typischerweise mit einem medizintechnischen Instrument durchgeführt, das in den Patienten eingeführt wird. Eine solche Intervention kann insbesondere ein bildüberwachtes Entnehmen einer Biopsie mittels einer Biopsienadel und/oder ein Ablatieren einer Läsion mittels eines Katheters sein. Dafür muss die Biopsienadel oder der Katheter in den Patienten eingeführt werden, während dieser für die medizinische Bildgebung in dem Patientenaufnahmebereich des Magnetresonanztomographens positioniert ist. Innerhalb des Patientenaufnahmebereiches ist eine Zugänglichkeit zu dem Patienten erschwert.

Es ist bekannt, das medizintechnische Instrument manuell in den Patienten einzuführen. Mit anderen Worten ist es bekannt, dass das medizintechnische Instrument durch ein medizinisches Personal in den Patienten eingeführt wird. Durch die erschwerte Zugänglichkeit muss das medizinische Personal dafür bestimmte körperliche Voraussetzungen mitbringen, beispielsweise ein Mindestgröße. Außerdem ist die Ergonomie beim Einführen des medizintechnischen Instrumentes für das medizinische Personal nicht gegeben.

Alternativ sind Interventionsroboter bekannt. Dabei bewegt sich der Roboter relativ zu dem Patienten in dem Patientenaufnahmebereich und führt dabei das medizintechnische Instrument in den Patienten ein. Allerdings ist es dafür notwendig, dass ausreichend Platz zwischen dem Patienten und einer dem Patienten zugewandten Innenwand des Patientenaufnahmebereiches ist. Da der Durchmesser des Patientenaufnahmebereiches typischerweise eher klein ist, ist diese Bedingung nicht für jeden Patienten erfüllt. Somit kann der Interventionsroboter nicht bei allen Patienten verwendet werden.

Es ist daher die Aufgabe der vorliegenden Erfindung, einen Magnetresonanztomographen bereitzustellen, der eine einfache Handhabung eines medizintechnischen Instrumentes bei einer bildgestützten Intervention innerhalb eines Patientenaufnahmebereiches ermöglicht.

Die Aufgabe wird gelöst durch einen Magnetresonanztomographen, ein Verfahren zum Planen einer Bewegung eines medizintechnischen Instrumentes in einem Patientenaufnahmebereich eines Magnetresonanztomographen, ein Planungssystem, welches ausgebildet ist, das Verfahren auszuführen, ein Computerprogrammprodukt, sowie ein computerlesbares Speichermedium gemäß den unabhängigen Ansprüchen. Vorteilhafte Weiterbildungen sind in den abhängigen Ansprüchen und in der folgenden Beschreibung aufgeführt.

Nachstehend wird die erfindungsgemäße Lösung der Aufgabe sowohl in Bezug auf die beanspruchten Vorrichtungen als auch in Bezug auf das beanspruchte Verfahren beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können die gegenständlichen Ansprüche (die beispielsweise auf eine Vorrichtung gerichtet sind) auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module ausgebildet.

Unabhängig vom grammatikalischen Geschlecht eines bestimmten Begriffes sind Personen mit männlicher, weiblicher oder anderer Geschlechteridentität mit umfasst.

Die Erfindung betrifft einen Magnetresonanztomographen umfassend einen Patiententisch, einen Patientenaufnahmebereich, eine Vorschubeinheit und ein Schienensystem. Die Vorschubeinheit ist dazu ausgebildet, ein medizintechnisches Instrument zu führen. Das Schienensystem verläuft innenumfänglich entlang einer Innenseite des Patientenaufnahmebereiches. Dabei ist der Patiententisch dazu ausgebildet, einen Patienten in dem Patientenaufnahmebereich zu positionieren. Dabei ist der Patiententisch derart bewegbar ausgebildet, dass das medizintechnische Instrument anhand einer Bewegung des Patiententisches in dem Patienten entlang einer Trajektorie geführt werden kann.

Der Magnetresonanztomograph umfasst bevorzugt einen medizinischen und/oder diagnostischen Magnetresonanztomographen, der zu einem Erfassen von medizinischen und/oder diagnostischen Bilddaten, insbesondere medizinischen und/oder diagnostischen Magnetresonanzbilddaten, eines Patienten ausgelegt bzw. ausgebildet ist. Der Magnetresonanztomograph umfasst hierzu eine Magneteinheit. Die Magneteinheit des Magnetresonanztomographen umfasst bevorzugt eine Detektoreinheit zur Erfassung der medizinischen und/oder diagnostischen Bilddaten. Vorteilhafterweise umfasst hierbei die Magneteinheit, einen Grundmagneten, eine Gradientenspuleneinheit und eine Hochfrequenzantenneneinheit. Die Hochfrequenzantenneneinheit ist fest innerhalb der Magneteinheit angeordnet und zur Aussendung eine Anregungspulses ausgelegt und/oder ausgebildet. Zur Erfassung der Magnetresonanzsignale weist der Magnetresonanztomograph lokale Hochfrequenzspulen auf, die um den zu untersuchenden Bereich des Patienten angeordnet werden.

Der Grundmagnet der Magneteinheit ist zur Erzeugung eines homogenen Grundmagnetfelds mit einer definierten bzw. bestimmten Magnetfeldstärke, wie beispielsweise mit einer definierten bzw. bestimmten Magnetfeldstärke von 3 T oder 1,5 T usw., ausgebildet. Insbesondere ist der Grundmagnet zur Erzeugung eines starken, konstanten und homogenen Grundmagnetfelds ausgebildet. Das homogene Grundmagnetfeld ist bevorzugt innerhalb des Patientenaufnahmebereichs des Magnetresonanztomographen angeordnet und/oder vorzufinden. Die Gradientenspuleneinheit ist zu einer Erzeugung von Magnetfeldgradienten, die für eine Ortskodierung während einer Bildgebung verwendet werden, ausgebildet.

Der Patientenaufnahmebereich ist zu einer Aufnahme des Patienten, insbesondere des zu untersuchenden Bereichs des Patienten, für eine medizinische Magnetresonanzuntersuchung ausgelegt bzw. ausgebildet. Beispielsweise ist hierzu der Patientenaufnahmebereich zylinderförmig ausgebildet bzw. zylinderförmig von der Magneteinheit umgeben. Dabei kann der Patientenaufnahmebereich einen kreisförmigen oder einen elliptischen Querschnitt aufweisen. Hierzu weist die Magneteinheit eine den Patientenaufnahmebereich zumindest teilweise umgebenden Umhausung der Gehäuseeinheit auf. Die den Patientenaufnahmebereich umgebende Umhausung kann hierbei auch einteilig bzw. einstückig mit der dem Patientenaufnahmebereich zugewandte Seite der Hochfrequenzantenneneinheit der Magneteinheit ausgebildet sein oder auch separat zur Hochfrequenzantenneneinheit der Magneteinheit ausgebildet sein.

Innerhalb des Patientenaufnahmebereichs ist bevorzugt ein Field of View (FOV) und/oder ein Isozentrum des Magnetresonanztomographen angeordnet. Das FOV umfasst bevorzugt einen Erfassungsbereich des Magnetresonanztomographen, innerhalb dessen die Bedingungen für eine Erfassung von medizinischen Bilddaten, insbesondere Magnetresonanzbilddaten, vorliegen, wie beispielsweise ein homogenes Grundmagnetfeld. Das Isozentrum des Magnetresonanztomographen umfasst bevorzugt den Bereich und/oder Punkt innerhalb des Magnetresonanztomographen, der die optimalen bzw. idealen Bedingungen für die Erfassung von medizinischen Bilddaten, insbesondere Magnetresonanzbilddaten, aufweist. Insbesondere umfasst das Isozentrum den homogensten Magnetfeldbereich innerhalb des Magnetresonanztomographen.

Der Magnetresonanztomograph umfasst eine Patientenlagerungsvorrichtung. Die Patientenlagerungsvorrichtung ist zu einer Positionierung und/oder Lagerung des Patienten für eine Magnetresonanzuntersuchung ausgebildet. Die Patientenlagerungsvorrichtung umfasst dabei den Patiententisch, der in den Patientenaufnahmebereich einfahrbar ausgebildet ist. Für eine Magnetresonanzuntersuchung wird der Patient derart auf dem Patiententisch positioniert, dass der zu untersuchende Bereich nach einem Positionieren des Patiententischs innerhalb des Patientenaufnahmebereichs innerhalb des Isozentrums des Patientenaufnahmebereichs angeordnet und/oder positioniert ist.

Für eine Kommunikation und/oder eines Informationsaustauschs des Patienten mit dem medizinischen Bedienpersonal während einer Magnetresonanzuntersuchung weist der Magnetresonanztomograph eine Kommunikationseinheit auf. Die Kommunikationseinheit weist auf Benutzerseite bevorzugt ein Kommunikationselement, wie beispielsweise eine Kommunikationskonsole zur Eingabe und/oder Ausgabe von Kommunikationsdaten, wie beispielsweise Informationen. Des Weiteren weist die Kommunikationseinheit auf Patientenseite ebenfalls zumindest ein Kommunikationselement auf. Insbesondere weist die Kommunikationseinheit ein visuelles oder auditives Kommunikationselement auf.

Das Schienensystem ist auf einer Innenseite des Patientenaufnahmebereiches angeordnet. Mit anderen Worten ist das Schienensystem auf einer dem Patienten während einer Untersuchung zugewandten Seite der Umhausung angeordnet. Das Schienensystem ist dabei innenumfänglich angeordnet. Mit anderen Worten ist das Schienensystem tangential zu einer Oberfläche der Umhausung angeordnet. Mit anderen Worten verläuft das Schienensystem wenigstens teilweise entlang eines Umfangs der Innenseite des Patientenaufnahmebereiches.

Die Vorschubeinheit ist dazu ausgebildet, das medizintechnische Instrument zu führen bzw. zu halten. Dafür kann das medizintechnische Instrument an der Vorschubeinheit befestigt werden. Die Vorschubeinheit kann dabei entlang des Schienensystems bewegt bzw. verschoben bzw. geführt werden. Insbesondere kann die Vorschubeinheit mithilfe von Rollen und/oder Lagern entlang des Schienensystem bewegt werden. Das Schienensystem gibt dabei eine Führung, insbesondere eine Richtung vor, in welche die Vorschubeinheit bewegbar ist.

Optional kann die Vorschubeinheit dazu ausgebildet sein, das medizintechnische Instrument zu kippen und/oder zu rotieren. Insbesondere kann das medizintechnische Instrument um eine Achse senkrecht zu dem Schienensystem rotierbar ausgebildet sein. Alternativ oder zusätzlich kann die Vorschubeinheit dazu ausgebildet sein, das medizintechnische Instrument relativ zu dieser Achse zu kippen.

Das medizintechnische Instrument kann insbesondere zum Durchführen einer medizinischen Intervention ausgebildet sein. Insbesondere kann das medizintechnische Instrument dazu ausgebildet sein, in einen Patienten eingeführt zu werden. Insbesondere kann das medizintechnische Instrument dazu ausgebildet sein, entlang einer Trajektorie in einem auf dem Patiententisch positionierten Patienten bewegt bzw. geführt zu werden.

Die Trajektorie kann dabei insbesondere gerade und/oder gekrümmt sein. Insbesondere kann die Trajektorie einen kürzesten Weg von einer Einstichstelle bzw. einem Eintrittspunkt des medizintechnischen Instrumentes in den Patienten zu einem Ort einer Intervention bzw. einer Zielposition in dem Patienten sein. Alternativ kann die Trajektorie gekrümmt sein. Alternativ kann die Trajektorie entlang bzw. innerhalb eines Blutgefäßes des Patienten verlaufen.

Die Intervention kann insbesondere eine Biopsie und/oder eine Ablation und/oder eine Katheterintervention sein. Das medizintechnische Instrument kann dabei insbesondere eine Biopsienadel oder ein Katheter oder eine hochintensive, fokussierte Ultraschallsonde umfassen.

Der Patiententisch ist dabei derart ausgebildet, das durch eine Bewegung des Patiententisches, das medizintechnische Instrument entlang der Trajektorie bewegt werden kann. Mit anderen Worten kann durch die Bewegung des Patiententisches der auf dem Patiententisch gelagerte Patient derart relativ zu der Vorschubeinheit und damit relativ zu dem an der Vorschubeinheit befestigten medizintechnischen Instrument bewegt werden, dass das medizintechnische Instrument entlang der Trajektorie in dem Patienten bewegt werden kann.

Der Erfinder hat erkannt, dass durch einen solchen Magnetresonanztomographen keine manuelle Bewegung des medizintechnischen Instrumentes durch das medizinische Personal notwendig ist. Auf diese Weise können die körperlichen Voraussetzung des medizinischen Personals weniger strikt ausgelegt werden. Außerdem werden die unergonomischen Situationen für das medizinische Personal vermieden. Der Erfinder hat außerdem erkannt, dass durch die Bewegung des Patienten relativ zu dem medizintechnischen Instrument, das Problem des zu geringen Platzes in dem Patientenaufnahmebereich überkommen werden kann. Insbesondere ist es somit nicht mehr notwendig, sehr viel Platz für das medizintechnische Instrument in dem Patientenaufnahmebereich vorzusehen, da es nicht notwendig ist, das medizintechnische Instrument selbst auf den Patienten zu zu bewegen und insbesondere in dem Patienten zu bewegen.

Nach einem Aspekt der Erfindung umfasst der Patiententisch wenigstens drei Achsen. Dabei sind die Achsen derart ausgebildet, dass das medizintechnische Instrument durch einen Bewegung des Patiententisches entlang der Trajektorie bewegbar ist. Dabei wird die Bewegung des Patiententisches durch eine Linearkombination einer Bewegung entlang der Achsen und/oder einer Rotation um wenigstens eine der Achsen erzeugt.

Der Patiententisch ist insbesondere entlang der drei Achsen bewegbar bzw. verschiebbar. Insbesondere ist der Patiententisch in dem Patientenaufnahmebereich entlang der drei Achsen bewegbar bzw. verschiebbar.

Außerdem kann der Patiententisch um wenigstens eine der drei Achsen rotierbar ausgebildet sein.

Die Achsen sind derart ausgerichtet, dass der Patient durch eine Bewegung des Patiententisches entlang oder eine Rotation um die Achsen relativ zu dem an der Vorschubeinheit angeordneten medizintechnischen Instrument derart bewegt werden kann, dass das medizintechnische Instrument die Trajektorie in dem Patienten abfahren kann bzw. entlang der Trajektorie bewegt bzw. geführt wird.

Der Erfinder hat erkannt, dass durch das Bewegen des Patienten relativ zu dem medizintechnischen Instrument mehr Platz für die Bewegung des medizintechnischen Instrumentes entlang der Trajektorie in dem Patientenaufnahmebereich bleibt, da kein großer Interventionsroboter zusätzlich in dem Patientenaufnahmebereich angeordnet werden muss, da die Bewegung durch den Patiententisch selbst erzeugt wird.

Nach einem weiteren Aspekt der Erfindung sind die drei Achsen derart ausgerichtet, dass sie einen dreidimensionalen Raum aufspannen.

Insbesondere können die Achsen dabei jeweils senkrecht zueinander ausgerichtet sein.

Insbesondere kann eine Achse parallel zur Schwerkraftrichtung ausgerichtet sein. Insbesondere können die anderen beiden Achsen dann senkrecht zur Schwerkraftrichtung ausgerichtet sein. Mit anderen Worten können zwei Achsen horizontal und eine Achse vertikal ausgerichtet sein.

Insbesondere kann eine der horizontalen Achsen entlang des Patientenaufnahmebereiches ausgerichtet sein. Mit anderen Worten ist dies Achse entlang einer Längsachse des Zylinders, der durch den Patientenaufnahmebereich geformt wird, ausgerichtet.

Insbesondere kann dann der Patiententisch wenigstens um die vertikale Achse drehbar bzw. rotierbar ausgebildet sein.

Der Erfinder hat erkannt, dass durch die beschriebene Anordnung der Achse, sichergestellt werden kann, dass der Patienten auf dem Patiententisch in jede mögliche Richtung gefahren bzw. bewegt werden kann. Auf diese Weise kann jede mögliche Trajektorie innerhalb des auf dem Patiententisch gelagerten Patienten mit dem medizintechnischen Instrument abgefahren werden.

Nach einem weiteren Aspekt der Erfindung ist das medizintechnische Instrument eine medizinische Nadel. Insbesondere ist das medizintechnische Instrument eine Biopsienadel.

Die medizinische Nadel ist dazu ausgebildet, in den Patienten eingeführt zu werden. Insbesondere kann die medizinische Nadel dazu ausgebildet sein, ein Medikament und/oder einen Wirkstoff durch die medizinische Nadel in den Patienten einzuführen. Alternativ oder zusätzlich kann die medizinische Nadel dazu ausgebildet sein, eine Probe, beispielsweise eine Gewebeprobe aus dem Patienten zu entnehmen. Dafür kann die medizinische Nadel insbesondere eine Biopsienadel sein. Eine Biopsienadel ist insbesondere zur Entnahme einer Biopsie ausgebildet.

Der Erfinder hat erkannt, dass besonders häufig bilgestützte Interventionen mit einer medizinischen Nadel in einem Magnetresonanztomographen durchgeführt werden. Der Erfinder hat erkannt, dass insbesondere eine Entnahme einer Biopsie durch die beschriebene Vorrichtung mit Verwendung einer Biopsienadel besonders einfach und besonders flexibel in einem Magnetresonanztomographen, bildgestützt durchgeführt werden kann.

Nach einem alternativen Aspekt der Erfindung ist das medizintechnische Instrument ein Katheter oder eine hochintensive, fokussierte Ultraschallsonde.

Der Katheter kann insbesondere dazu ausgebildet sein, einen Stent einzuführen oder eine Ballondilatation eines Blutgefä-βes durchzuführen oder eine Ablation durchzuführen.

Die hochintensive, fokussierte Ultraschallsonde kann insbesondere mit einem Katheter geführt werden. Die hochfokussierte Ultraschallsonde kann dabei insbesondere zum Aussenden hochintensiver, fokussierter Ultraschallpulse ausgebildet sein. Diese Ultraschallpulse können dann insbesondere dazu ausgebildet sein, um eine Ablation durchzuführen.

Der Erfinder hat erkannt, das mit dem beschriebenen Magnetresonanztomographen beliebige Interventionen durchführbar sind, bei welchen ein beliebiges medizintechnisches Instrument in einen Patienten eingeführt wird. Der Erfinder hat erkannt, dass durch die Bewegung des Patiententisches für verschiedene medizintechnische Instrumente Trajektorien abgefahren werden können.

Nach einem weiteren Aspekt der Erfindung verläuft das Schienensystem entlang eines Kreisbogens.

Insbesondere ist der Querschnitt des Patientenaufnahmebereiches dabei kreisförmig. Das Schienensystem verläuft dabei entlang eines Umfangs des Querschnittes. Das Schienensystem verläuft dabei entlang eines Kreisbogens. Der Kreisbogen kann dabei 30° oder 60° oder 90° oder 120° oder 150° oder bevorzugt 180° oder 210° oder 240° oder bevorzugt 270° oder 300° oder 330° eines Umfangs eines Kreises umfassen.

Der Erfinder hat erkannt, dass entlang des Schienensystems die Vorschubeinheit mit dem medizintechnischen Instrument um den auf dem Patiententisch positionierten Patienten in dem Patientenaufnahmebereich herumgefahren werden kann. Der Erfinder hat erkannt, dass auf diese Weise das medizintechnische Instrument entlang des Schienensystems mittels der Vorschubeinheit zu einem Einstichpunkt am Patienten gefahren werden kann. An dem Einstichpunkt soll das medizintechnische Instrument in den Patienten eingeführt werden. Der Erfinder hat erkannt, dass durch die Bewegung des Patiententisches und das Verfahren des medizintechnischen Instrumentes entlang des Schienensystems das medizintechnische Instrument entlang der Trajektorie bewegt werden kann.

Nach einem weiteren Aspekt der Erfindung ist das Schienensystem ringförmig ausgebildet.

Dabei bildet das Schienensystem einen geschlossenen Ring entlang des eines inneren Umfangs der Innenseite bzw. der Patienten zugewandten Seite des Patientenaufnahmebereiches. Insbesondere kann dabei der Patientenaufnahmebereich einen kreisförmigen Querschnitt aufweisen.

Der Erfinder hat erkannt, dass dann das medizintechnische Instrument mittels der Vorschubeinheit entlang des Schienensystems um den kompletten Umfang des Inneren des Patientenaufnahmebereiches verfahren werden kann. Der Erfinder hat erkannt, dass auf diese Weise immer der kürzeste Weg entlang des Schienensystems zu einem Einstichpunkt, an dem das medizintechnische Instrument in den Patienten eingeführt werden soll, zurückgelegt werden kann. Der Erfinder hat erkannt, dass auf diese Weise eine Vorbereitung und Durchführung der Intervention beschleunigt werden kann. Insbesondere kann auf diese Weise ein Patientenkomfort erhöht werden, da der Patient eine möglichst kurze Wartezeit hat.

Nach einem weiteren Aspekt der Erfindung umfasst der Magnetresonanztomograph eine Magneteinheit. Dabei wird der Patientenaufnahmebereich von der Magneteinheit umschlossen. Dabei bildet die Magneteinheit einen innenumfänglichen Spalt, in welchem das Schienensystem angeordnet ist.

Die Magneteinheit ist dabei wie oben beschrieben ausgebildet. Die Magneteinheit ist dabei insbesondere zylinderförmig ausgebildet. Der Zylinder kann dabei einen kreisförmigen oder einen elliptischen Querschnitt aufweisen. Der Patientenaufnahmebereich ist dabei im Inneren der Magneteinheit angeordnet. Mit anderen Worten wird der Patientenaufnahmebereich von der Magneteinheit umschlossen.

Die Magneteinheit ist dabei zumindest teilweise in zwei Teilstücken unterteilt, die einen innenumfänglich Spalt bilden. Mit anderen Worten ist die Magneteinheit zumindest teilweise entlang eines Querschnitts der Magneteinheit in die zwei Teilstücken unterteilt. Die beiden Teilstücken können miteinander verbunden sein. Alternativ können die zwei Teilstücken unabhängig voneinander sein. Insbesondere ist dabei die von der Magneteinheit umfasste Gradientenspuleneinheit in zwei Teilstücke unterteilt und den Spalt bilden. Insbesondere kann außerdem die von der Magneteinheit umfasste Hochfrequenzantenneneinheit in zwei Teilstücke unterteilt sein und den Spalt bilden. Insbesondere kann in Ausführungen der Erfindung der von der Magneteinheit umfasste Grundmagnet weiterhin einstückig ausgebildet sein. Mit anderen Worten kann der Grundmagnet in Ausführungen keinen Spalt bilden.

Der Spalt ist dabei entlang eines Umfangs der Innenseite der Magneteinheit angeordnet.

Der Spalt ist derart ausgebildet, dass das Schienensystem in dem Spalt angeordnet ist. Das Schienensystem ist damit zwischen den beiden Teilstücken der Magneteinheit angeordnet. Das Schienensystem ist damit in die Magneteinheit integriert.

Der Erfinder hat erkannt, dass auf diese Weise zusätzlicher Platz im Patientenaufnahmebereich generiert werden kann. Insbesondere kann auf diese Weise vermieden werden, dass das Schienensystem zusätzlichen Platz in dem Patientenaufnahmebereich benötigt. Mit anderen Worten kann der Platz in dem Patientenaufnahmebereich, der durch das Schienensystem, die Vorschubeinheit und das medizintechnische Instrument benötigt wird, auf diese Weise minimiert werden.

Nach einem weiteren Aspekt der Erfindung ist die Vorschubeinheit dazu ausgebildet, das medizintechnische Instrument senkrecht zu dem Schienensystem zu bewegen.

Insbesondere ist die Vorschubeinheit dazu ausgebildet, das medizintechnische Instrument auf den Patienten zu zu bewegen. Insbesondere ist die Vorschubeinheit dazu ausgebildet, das medizintechnische Instrument radial nach innen in den Patientenaufnahmebereich zu bewegen. Insbesondere ist die Vorschubeinheit auf diese Weise dazu ausgebildet, das medizintechnische Instrument in den Patienten einzuführen bzw. einzustechen.

Der Erfinder hat erkannt, dass mittels der Vorschubeinheit besonders genaue Bewegungen des medizintechnischen Instrumentes realisiert werden können. Der Erfinder hat erkannt, dass dies insbesondere bei einem Vorschub bzw. Einstechen des medizintechnischen Instrumentes relevant ist. Der Erfinder hat erkannt, dass die radiale Bewegung durch die Vorschubeinheit die Bewegung durch den Patiententisch ergänzen kann und, dass das medizintechnische Instrument auf diese Weise sehr präzise entlang der Trajektorie geführt werden kann.

Nach einem weiteren Aspekt der Erfindung ist die Vorschubeinheit dazu ausgebildet das medizintechnische Instrument zu drehen und/oder zu verschwenken.

Insbesondere kann die Vorschubeinheit das medizintechnische Instrument um eine radiale Achse verdrehen und/oder verschwenken. Die radiale Achse ist dabei radial in das Innere des Patientenaufnahmebereiches ausgerichtet. Insbesondere ist die radiale Achse senkrecht zu dem Schienensystem ausgerichtet.

Beim Verschwenken kann das medizintechnische Instrument derart verstellt werden, dass es einen Winkel mit der radialen Achse ausbildet. Insbesondere kann das medizintechnische Instrument durch das Verschwenken einen Winkel zwischen 0° und 90° mit der radialen Achse einschließen. Insbesondere kann das medizintechnische Instrument einen Winkel von 0° mit der radialen Achse einschließen. Dann ist das medizintechnische Instrument in Richtung eines Zentrums des Patientenaufnahmebereiches und insbesondere senkrecht zu dem Schienensystem ausgerichtet. Der Winkel, um den das medizintechnische Instrument maximal mittels der Vorschubeinheit verschwenkt werden kann ist dabei kleiner als 90°.

Beim Verdrehen wird das medizintechnische Instrument um die radiale Achse gedreht. Insbesondere kann das verschwenkte medizintechnische Instrument um die radiale Achse gedreht werden.

Der Erfinder hat erkannt, dass durch das Verschwenken und/oder Verdrehen des medizintechnischen Instrumentes sichergestellt werden kann, dass das medizintechnische Instrument in eine richtige Richtung ausgerichtet ist. Mit anderen Worten kann durch das Verdrehen und/oder Verschwenken sichergestellt werden, dass das medizintechnische Instrument in die Richtung der Trajektorie ausgerichtet ist. Mit anderen Worten kann auf diese Weise sichergestellt werden, dass beim Einführen bzw. Einstechen des medizintechnischen Instrumentes in den Patienten das medizintechnische Instrument in Richtung der Trajektorie ausgerichtet ist.

Nach einem weiteren Aspekt der Erfindung umfasst die Vorschubeinheit wenigstens einen Piezomotor. Der Piezomotor ist dazu ausgebildet, das medizintechnische Instrument zu bewegen.

Insbesondere ist der Piezomotor dazu ausgebildet, das medizintechnische Instrument, wie oben beschrieben, senkrecht zu dem Schienensystem zu bewegen. Alternativ oder zusätzlich ist der Piezomotor dazu ausgebildet, das medizintechnische Instrument zu verschwenken und/oder zu verdrehen.

Der Erfinder hat erkannt, dass ein Piezomotor präzise genug ist, um die Bewegungen des medizintechnischen Instrumentes entlang der Trajektorie auszuführen bzw. zu koordinieren. Insbesondere ist der Piezomotor präzise genug, das medizintechnische Instrument bei einer Intervention zu führen.

Nach einem weiteren Aspekt der Erfindung umfasst die Vorschubeinheit ein Auslösesystem. Das Auslösesystem ist dazu ausgebildet, das medizintechnische Instrument von der Vorschubeinheit zu lösen, wenn eine auf das medizintechnische Instrument wirkende Kraft einen Grenzwert überschreitet.

Das Auslösesystem umfasst dabei insbesondere einen Kraftsensor. Der Kraftsensor ist dazu ausgebildet, eine auf das medizintechnische Instrument wirkende Kraft zu erfassen. Insbesondere ist das Kraftsensor dazu ausgebildet, eine Kraft zu erfassen, die seitlich auf das medizintechnische Instrument wirkt. Mit anderen Worten ist der Kraftsensor dazu ausgebildet, eine Kraft zu erfassen, die nicht entlang der Trajektorie wirkt.

Das Auslösesystem kann einen Betrag der gemessenen Kraft mit dem Grenzwert vergleichen. Insbesondere kann das Auslösesystem einen Betrag eines senkrecht zu der Trajektorie auf das medizintechnische Instrument wirkenden Beitrags der Kraft mit dem Grenzwert vergleichen.

Der Grenzwert ist insbesondere vorbestimmt bzw. vorgegeben.

Das Auslösesystem kann eine Verbindung zwischen der Vorschubeinheit und dem medizintechnischen Instrument lösen bzw. trennen.

Insbesondere kann nach dem Lösen bzw. Trennen der Verbindung das medizintechnische Instrument nicht mehr mit der Vorschubeinheit geführt bzw. bewegt werden. Insbesondere ist das medizintechnischen Instrument nach dem Lösen bzw. Trennen der Verbindung nicht mehr fest mit der Vorschubeinheit verbunden. Somit führt nach dem Lösen bzw. Trennen eine relative Bewegung des Patiententisches zu der Vorschubeinheit nicht mehr zu einer relativen Bewegung des medizintechnischen Instrumentes relativ zu dem Patiententisch.

Der Erfinder hat erkannt, dass das Auslösesystem zum Schutz des Patienten geeignet ist. Insbesondere kann, wenn eine ungewollte Bewegung durch den Patienten, den Patiententisch, das Schienensystem und/oder die Vorschubeinheit erfolgt, durch das Auslösesystem eine Verletzung des Patienten verhindert werden. Es kann insbesondere verhindert werden, dass das medizintechnische Instrument abseits der Trajektorie in dem Patienten bewegt wird.

Die Erfindung betrifft außerdem ein computerimplementiertes Verfahren zum Planen einer Bewegung eines medizintechnischen Instrumentes in einem Patientenaufnahmebereich eines oben beschriebenen Magnetresonanztomographen entlang einer Trajektorie. Das Verfahren umfasst einen Verfahrensschritt eines Empfangens einer Trajektorie. Dabei gibt die Trajektorie relativ zu einem Patienten vor, wie das medizintechnische Instrument geführt werden soll. Das Verfahren umfasst weiter einen Verfahrensschritt eines Bestimmens von wenigstens einem Tischparameter einer Tischbewegung des Patiententisches in Abhängigkeit der Trajektorie. Das Verfahren umfasst weiter einen Verfahrensschritt eines Bestimmens von wenigstens einem Schienenparameter einer Bewegung des medizintechnischen Instrumentes entlang des Schienensystems in Abhängigkeit der Trajektorie. Dabei sind der wenigstens eine Tischparameter und der wenigstens eine Schienenparameter derart ausgebildet, dass das medizintechnische Instrument in einem auf dem Patiententisch gelagerten Patienten entlang der Trajektorie mittels der Tischbewegung und der Bewegung des medizintechnischen Instrumentes entlang des Schienensystems geführt werden kann. Das Verfahren umfasst außerdem einen Verfahrensschritt eines Bereitstellens des wenigstens einen Tischparameters der Tischbewegung und des wenigstens einen Schienenparameters der Bewegung des medizintechnischen Instrumentes entlang des Schienensystems.

Das Verfahren ist insbesondere dazu ausgebildet, eine Bewegung des medizintechnischen Instrumente entlang der Trajektorie zu planen. Insbesondere ist das Verfahren dazu ausgebildet, Parameter, insbesondere wenigstens einen Tischparameter und einen Schienenparameter bereitzustellen, mit welchen das medizintechnische Instrument entlang der Trajektorie in einem Patienten bewegt werden kann bzw. geführt werden kann. Insbesondere kann das medizintechnische Instrument dabei durch eine Kombination aus einer Tischbewegung des Patiententisches und einer Bewegung des medizintechnischen Instrumentes entlang des Schienensystems entlang der Trajektorie bewegt bzw. geführt werden.

Der Magnetresonanztomograph mit dem Patientenaufnahmebereich ist dabei wie oben beschrieben ausgebildet. Das medizintechnische Instrument ist dabei wie oben beschrieben ausgebildet.

Die Trajektorie wird dabei mittels einer Schnittstelle empfangen. Die Trajektorie ist dabei wie oben beschrieben ausgebildet. Die Trajektorie gibt dabei an, entlang welches Weges das medizintechnische Instrument in einem Patienten von einer Einstichstelle bzw. Eintrittspunkt zu einem Ort einer Intervention bzw. Zielort bewegt bzw. geführt werden soll.

Der wenigstens eine Tischparameter wird mittels einer Recheneinheit bestimmt. Der Tischparameter gibt dabei eine Bewegung des Patiententisches vor, um das medizintechnische Instrument entlang der Trajektorie zu bewegen bzw. zu führen. Der Begriff "Tischbewegung" beschreibt dabei eine Bewegung des Patiententisches. Der Patiententisch ist dabei wie oben beschrieben ausgebildet. Insbesondere kann der Patiententisch wie oben beschrieben entlang drei Achsen verschiebbar und/oder rotierbar sein. Die drei Achsen spannen dabei vorteilhafterweise einen dreidimensionalen Raum auf. Die drei Achsen sind dabei vorteilhafterweise wie oben beschrieben ausgerichtet.

Der wenigstens eine Schienenparameter wird mittels der Recheneinheit bestimmt. Das medizintechnische Instrument ist mit einer wie oben beschrieben ausgebildeten Vorschubeinheit mit dem wie oben beschrieben ausgebildeten Schienensystem verbunden bzw. gekoppelt bzw. an dem Schienensystem befestigt. Durch Verschieben bzw. Bewegen der Vorschubeinheit entlang des Schienensystems kann die Position des medizintechnischen Instrumentes variiert werden. Wie weit bzw. wohin entlang des Schienensystems das medizintechnische Instrument entlang des Schienensystems verschoben werden soll, kann insbesondere mittels des wenigstens einen Schienenparameters vorgegeben werden. Insbesondere kann durch den Schienenparameter die Einstichstelle vorgegeben werden.

Der wenigstens eine Tischparameter und der wenigstens eine Schienenparameter werden dabei derart bestimmt, dass das medizintechnische Instrument durch eine auf dem Tischparameter basierende Bewegung des Patiententisches und eine auf dem Schienenparameter basierende Bewegung des medizintechnischen Instrumentes entlang des Schienensystems entlang der Trajektorie in einem auf dem Patiententisch gelagerten Patienten in dem Patientenaufnahmebereich bewegt bzw. geführt werden kann.

In dem Verfahrensschritt des Bereitstellens des wenigstens einen Tischparameters und des wenigstens einen Schienenparameters werden der wenigstens eine Tischparameter und der wenigstens eine Schienenparameter mittels der Schnittstelle bereitgestellt. Insbesondere können der wenigstens einen Tischparameter und der wenigstens eine Schienenparameter einem oben beschriebenen Magnetresonanztomographen bereitgestellt werden. Alternativ können der wenigstens eine Tischparameter und der wenigstens eine Schienenparameter einer Datenbank bereitgestellt werden.

Der Erfinder hat erkannt, dass für einen wie oben beschriebenen ausgebildeten Magnetresonanztomographen Parameter, insbesondere wenigstens ein Tischparameter und wenigstens ein Schienenparameter bestimmt werden können, welche eine Bewegung des medizintechnischen Instrumentes entlang der Trajektorie realisieren können. Der Erfinder hat erkannt, dass die Bewegung entlang der Trajektorie durch eine Kombination der Bewegung des Patiententisches und der Bewegung des medizintechnischen Instrumentes entlang des Schienensystems möglich ist.

Nach einem optionalen Aspekt der Erfindung wird beim Bestimmen des wenigstens einen Tischparameters und beim Bestimmen des wenigstens einen Schienenparameters eine Positionierung des Patienten auf dem Patiententisch in dem Patientenaufnahmebereich berücksichtigt.

Insbesondere können die Parameter (der Tischparameter und der Schienenparameter) zunächst basierend auf einer idealen Positionierung des Patienten bestimmt werden. Anschließend können die Parameter basierend auf einer realen Positionierung des Patienten angepasst werden, sobald diese bekannt ist. Insbesondere können dabei auch alle weiteren, im Folgenden beschriebenen Parameter zur Bewegung des medizintechnischen Instrumentes entlang der Trajektorie angepasst werden.

Der Erfinder hat erkannt, dass beim Planen zunächst von einer idealen Positionierung des Patienten auf dem Patiententisch ausgegangen werden kann. Der Erfinder hat erkannt, dass die Parameter leicht an die reale, tatsächliche Positionierung angepasst werden kann, sobald diese bekannt ist. Der Erfinder hat erkannt, dass auf diese Weise beispielsweise eine leichte Schieflage und/oder Verdrehung des Patienten auf dem Patiententisch berücksichtigt werden kann.

Nach einem Aspekt der Erfindung umfasst das Verfahren außerdem einen Verfahrensschritt eines Bestimmens wenigstens eines Atemparameters der Tischbewegung. Dabei ist der Atemparameter dazu ausgebildet, den Patiententisch zur Kompensation einer Atembewegung des Patienten zu bewegen. Das Verfahren umfasst außerdem einen Verfahrensschritt eines Bereitstellens des wenigstens einen Atemparameters der Tischbewegung.

Der wenigstens eine Atemparameter gibt insbesondere eine periodische, zu einer Atmung eines auf dem Patiententisch positionierten Patienten synchrone Tischbewegung vor. Der Atemparameter ist dabei derart ausgebildet, dass sich eine Spitze des medizintechnischen Instrumentes in dem Patienten nicht aufgrund einer Atmung des Patienten relativ zu dem Patienten bewegt und dabei von der Trajektorie abweicht.

Die Spitze des medizintechnischen Instrumentes ist dabei der Teil der medizintechnischen Instrumentes, der am weitesten von der Verbindung zwischen der Vorschubeinheit und dem medizintechnischen Instrument entfernt ist.

Beim Bestimmen des wenigstens einen Atemparameters können insbesondere Sensordaten, von wenigstens einem Sensor, der die Atembewegung des Patienten erfasst, berücksichtigt werden. Der wenigstens eine Sensor kann dabei auf dem Patienten angeordnet sein und/oder in dem Patiententisch integriert sein.

Alternativ kann beim Bestimmen des wenigstens einen Atemparameters eine Atembewegung des Patienten abgeschätzt werden. Insbesondere kann die Atembewegung bei einem intubierten Patienten in Abhängigkeit von der Beatmung abgeschätzt werden.

Der wenigstens eine Atemparameter wird mittels der Schnittstelle bereitgestellt. Insbesondere wird der Atemparameter analog zu dem Tischparameter und dem Schienenparameter bereitgestellt.

Der Erfinder hat erkannt, dass durch eine periodische Tischbewegung eine Atembewegung des Patienten kompensiert werden kann. Der Erfinder hat erkannt, dass auf diese Weise eine Sicherheit für den Patienten erhöht werden kann, das vermieden werden kann, dass das medizintechnische Instrument aufgrund einer Atembewegung an einen Ort innerhalb des Patienten bewegt wird, an welchen es nicht bewegt werden sollte und den Patienten verletzt.

Nach einem weiteren Aspekt der Erfindung umfasst das Verfahren außerdem einen Verfahrensschritt eines Bestimmens wenigstens eines Vorschubparameters in Abhängigkeit der Trajektorie. Dabei ist die Vorschubeinheit dazu ausgebildet mittels des wenigstens einen Vorschubparameters das medizintechnische Instrument in Kombination mit der Tischbewegung des Patiententisches und der Bewegung des medizintechnischen Instrumentes entlang des Schienensystems entlang der Trajektorie zu führen. Das Verfahren umfasst außerdem einen Verfahrensschritt eines Bereitstellens des wenigstens einen Vorschubparameters.

Der wenigstens eine Vorschubparameter wird dabei mittels der Recheneinheit bestimmt. Die Vorschubeinheit ist dabei wie oben beschrieben dazu ausgebildet, das medizintechnische Instrument senkrecht zu dem Schienensystem zu bewegen bzw. zu führen. Diese Bewegung wird durch den Vorschubparameter vorgegeben bzw. beschrieben.

Der wenigstens eine Vorschubparameter wird analog zu dem wenigstens einen Tischparameter und dem wenigstens einen Schienenparameter mittels der Schnittstelle bereitgestellt.

Der Erfinder hat erkannt, dass die gesamte Bewegung parametrisiert werden kann. Der Erfinder hat erkannt, dass das medizintechnische Instrument durch eine Kombination aus einer Bewegung des Patiententisches, der Bewegung des medizintechnischen Instrumentes entlang des Schienensystems und einem Vorschub des medizintechnischen Instrumentes durch die Vorschubeinheit entlang der Trajektorie bewegt werden kann.

Nach einem optionalen Aspekt der Erfindung umfasst das Verfahren einen weiteren Verfahrensschritt eines Bestimmens wenigstens eines Drehparameters und/oder eines Verschwenkparameters. Dabei ist die Vorschubeinheit dazu ausgebildet, das medizintechnische Instrument basierend auf dem wenigstens einen Drehparameter zu drehen und/oder basierend auf dem wenigstens einen Verschwenkparameter zu verschwenken.

Das Verschwenken und/oder Drehen ist dabei wie oben beschrieben ausgebildet. Der wenigstens eine Drehparameter und/oder der wenigstens eine Verschwenkparameter sind dazu ausgebildet, eine Ausrichtung des medizintechnischen Instrumentes mittels der Vorschubeinheit einzustellen. Dabei ist die Ausrichtung derart ausgebildet, dass das medizintechnische Instrument entlang der zw. tangential zu der Trajektorie ausgerichtet ist. Insbesondere kann die Ausrichtung mittels des wenigstens einen Drehparameters und/oder des wenigstens einen Verschwenkparameters während der Bewegung mittels dem wenigstens einen Tischparameter und dem wenigstens einen Schienenparameter und optional dem wenigstens einen Vorschubparameter korrigiert bzw. angepasst werden.

Der Erfinder hat erkannt, dass die Bewegung des medizintechnischen Instrumentes im Vorfeld geplant werden kann. Der Erfinder hat erkannt, dass dafür auch ein Drehen und/oder Schwenken des medizintechnischen Instrumentes mittels der Vorschubeinheit notwendig sein kann.

Nach einem weiteren optionalen Aspekt der Erfindung umfasst das Verfahren außerdem einen Verfahrensschritt eines Steuerns des Magnetresonanztomographen in Abhängigkeit des wenigstens einen Tischparameters und des wenigstens einen Schienenparameters und optional auch in Abhängigkeit des wenigstens einen Atemparameters und/oder des wenigstens einen Vorschubparameters.

Insbesondere werden die genannten Parameter dann beim Bereitstellen dem Magnetresonanztomographen bereitgestellt. Insbesondere kann dann der Magnetresonanztomograph basierend auf dem wenigstens einen Tischparameter und optional basierend auf dem wenigstens einen Atemparameter eine Bewegung des Patententisches veranlassen bzw. ausführen. Außerdem kann der Magnetresonanztomograph basierend auf dem wenigstens einen Schienenparameter eine Bewegung der Vorschubeinheit und damit des medizintechnischen Instrumentes entlang des Schienensystems veranlassen. Optional kann der Magnetresonanztomograph basierend auf dem wenigstens einen Vorschubparameter eine Bewegung des medizintechnischen Instrumentes mittels der Vorschubeinheit veranlassen. Dabei kann das medizintechnische Instrument insbesondere mittels der Vorschubeinheit senkrecht zu dem Schienensystem bewegt werden. Alternativ oder zusätzlich kann das medizintechnische Instrument durch die Vorschubeinheit in Abhängigkeit von dem wenigstens einen Vorschubparameter um eine Achse senkrecht zu dem Schienensystem gedreht und/oder verschwenkt werden.

Durch die beschriebenen Bewegungen des Magnetresonanztomographen kann das medizintechnische Instrument entlang einer Trajektorie in einem auf dem Patiententisch positionierten Patienten bewegt werden. Insbesondere können die entsprechenden Bewegungen auch ausgeführt werden, ohne, dass ein Patient auf dem Patiententisch positioniert ist.

Der Erfinder hat erkannt, dass mit dem beschriebenen Verfahren Parameter bestimmt werden können, mittels derer der Magnetresonanztomograph derart gesteuert werden kann, dass das medizintechnische Instrument entlang der Trajektorie bewegt werden kann. Insbesondere ist dafür weder eine manuelle Bewegung des medizintechnischen Instrumentes noch eine Bewegung mittels eines Interventionsroboters notwendig. Dies führt zu einer einfacheren Handhabung und leichteren Durchführung der Intervention. Insbesondere können auf diese Weise die körperlichen Voraussetzungen an das medizinische Personal reduziert werden. Insbesondere wird durch die Bewegung des Pateinten auf dem Patiententisch der Platzbedarf für die Bewegung des medizintechnischen Instrumentes in dem Patientenaufnahmebereich reduziert. Auf diese Weise kann auch an größeren Patienten eine Intervention mittels dem medizintechnischen Instrument in dem Magnetresonanztomographen durchgeführt werden.

Die Erfindung betrifft außerdem ein Planungssystem zum Planen einer Bewegung eine medizintechnischen Instrumentes in einem Patientenaufnahmebereich eines oben beschriebenen Magnetresonanztomographen entlang einer Trajektorie. Das Planungssystem umfasst dabei eine Schnittstelle und eine Recheneinheit, die zum Ausführen der folgenden Verfahrensschritte ausgebildet sind:
- Empfangen einer Trajektorie,
   wobei die Trajektorie relativ zu einem Patienten vorgibt, wie das medizintechnische Instrument geführt werden soll,
- Bestimmen von wenigstens einem Tischparameter einer Tischbewegung des Patiententisches,
- Bestimmen von wenigstens einem Schienenparameters einer Bewegung des medizintechnischen Instrumentes entlang des Schienensystems,
   wobei der wenigstens eine Tischparameter und der wenigstens eine Schienenparameter derart ausgebildet sind, dass das medizintechnische Instrument in einem auf dem Patiententisch gelagerten Patienten entlang der Trajektorie mittels der Tischbewegung und der Bewegung des medizintechnischen Instrumentes geführt wird,
- Bereitstellen des wenigstens einen Tischparameters der Tischbewegung und des wenigstens einen Schienenparameters der Bewegung des medizintechnischen Instrumentes entlang des Schienensystems.

Eine solches Planungssystem kann insbesondere dazu ausgebildet sein das zuvor beschriebene Verfahren zum Planen einer Bewegung eine medizintechnischen Instrumentes in einem Patientenaufnahmebereich eines oben beschriebenen Magnetresonanztomographen entlang einer Trajektorie und seine Aspekte auszuführen. Das Planungssystem ist dazu ausgebildet dieses Verfahren und seine Aspekte auszuführen, indem die Schnittstelle und die Recheneinheit ausgebildet sind, die entsprechenden Verfahrensschritte auszuführen.

Die Erfindung betrifft auch ein Computerprogrammprodukt mit einem Computerprogramm sowie ein computerlesbares Medium. Eine weitgehend softwaremäßige Realisierung hat den Vorteil, dass auch schon bisher verwendete Planungssystems auf einfache Weise durch ein Software-Update nachgerüstet werden können, um auf die beschriebene Weise zu arbeiten. Ein solches Computerprogrammprodukt kann neben dem Computerprogramm gegebenenfalls zusätzliche Bestandteile wie z. B. eine Dokumentation und/oder zusätzliche Komponenten, sowie Hardware-Komponenten, wie z.B. Hardware-Schlüssel (Dongles etc.) zur Nutzung der Software, umfassen.

Insbesondere betrifft die Erfindung auch ein Computerprogrammprodukt mit einem Computerprogramm, welches direkt in einen Speicher einer Planungssystems ladbar ist, mit Programmabschnitten, um alle Schritte des oben beschriebenen Verfahrens zum Planen einer Bewegung eine medizintechnischen Instrumentes in einem Patientenaufnahmebereich eines oben beschriebenen Magnetresonanztomographen entlang einer Trajektorie und seine Aspekte auszuführen, wenn die Programmabschnitte von dem Planungssystem ausgeführt werden.

Insbesondere betrifft die Erfindung ein computerlesbares Speichermedium, auf welchem von einem Planungssystem lesbare und ausführbare Programmabschnitte gespeichert sind, um alle Schritte des oben beschriebenen Verfahrens zum Planen einer Bewegung eine medizintechnischen Instrumentes in einem Patientenaufnahmebereich eines oben beschriebenen Magnetresonanztomographen entlang einer Trajektorie und seine Aspekte auszuführen, wenn die Programmabschnitte von dem Planungssystem ausgeführt werden.

Die oben beschriebenen Eigenschaften, Merkmale und Vorteile dieser Erfindung werden klarer und verständlicher im Zusammenhang mit folgenden Figuren und ihren Beschreibungen. Dabei sollen die Figuren und Beschreibungen die Erfindung und ihre Ausführungsformen in keiner Weise einschränken.

In verschiedenen Figuren sind gleiche Komponenten mit korrespondierenden Bezugszeichen versehen. Die Figuren sind in der Regel nicht maßstabsgetreu.

Es zeigen
Fig. 1 ein Ausführungsbeispiel eines Magnetresonanztomographen,
Fig. 2 ein Ausführungsbeispiel eines computerimplementierten Verfahrens zum Planen einer Bewegung eines medizintechnischen Instrumentes in einem Patientenaufnahmebereich eines Magnetresonanztomographen entlang einer Trajektorie,
Fig. 3 ein Ausführungsbeispiel eines Planungssystems zum Planen einer Bewegung eines medizintechnischen Instrumentes in einem Patientenaufnahmebereich eines Magnetresonanztomographen entlang einer Trajektorie.

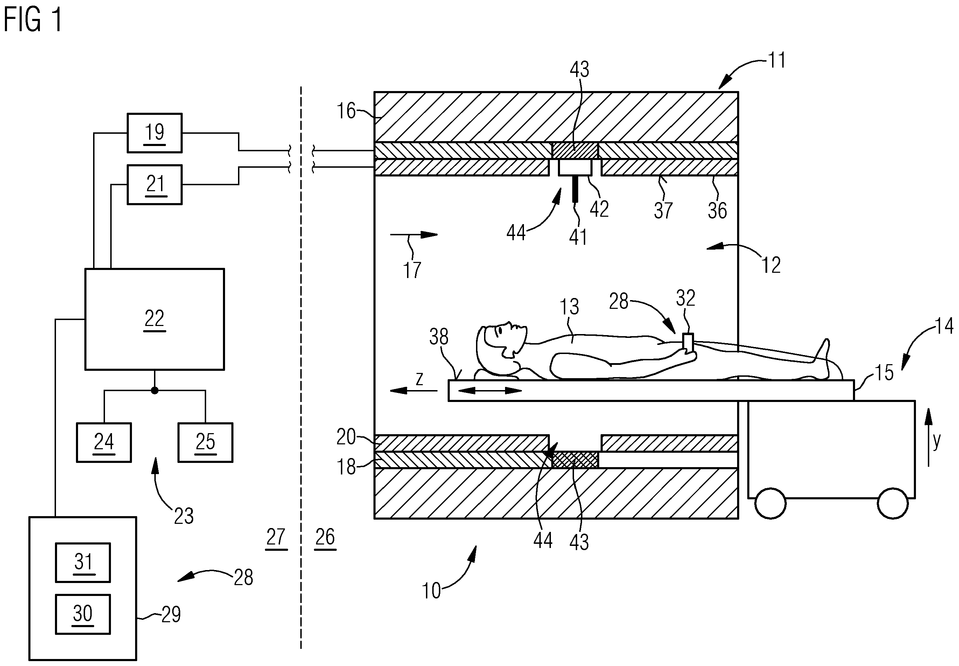

**Figur 1** zeigt ein Ausführungsbeispiel eines Magnetresonanztomographen 10.

Der Magnetresonanztomograph 10 umfasst eine Magneteinheit 11. Zudem weist der Magnetresonanztomograph 10 einen Patientenaufnahmebereich 12 auf, welcher zu einer Aufnahme eines Patienten 13 ausgebildet ist. Der Patientenaufnahmebereich 12 im vorliegenden Ausführungsbeispiel ist zylinderförmig ausgebildet und in einer Umfangsrichtung von der Magneteinheit 11 zylinderförmig umgeben. Grundsätzlich ist jedoch eine davon abweichende Ausbildung des Patientenaufnahmebereichs 12 jederzeit denkbar. Der Patient 13 kann mittels einer Patientenlagerungsvorrichtung 14 des Magnetresonanztomograph 10 in den Patientenaufnahmebereich 12 geschoben und/oder gefahren werden. Die Patientenlagerungsvorrichtung 14 weist hierzu einen innerhalb des Patientenaufnahmebereichs 12 bewegbar ausgestalteten Patiententisch 15 auf. Insbesondere ist hierbei der Patiententisch 15 in Richtung einer Längserstreckung des Patientenaufnahmebereichs 12 und/oder in z-Richtung bewegbar gelagert.

Der Patiententisch 15 umfasst in Ausführungen der Erfindung wenigstens drei Achsen. Die Achsen sind dabei derart ausgebildet, dass der Patiententisch 15 durch eine Linearkombination einer Bewegung entlang und/oder einer Rotation um die Achsen bewegt werden kann.

Der Patiententisch 15 ist insbesondere außerdem in y-Richtung und in x-Richtung bewegbar gelagert. Die x-Richtung und die y-Richtung stehen jeweils senkrecht zu der z-Richtung. Außerdem sind x-Richtung und y-Richtung senkrecht zueinander ausgerichtet. Die x-, die y- und die z-Richtung definieren dabei die Achsen, entlang welchen der Patiententisch 15 bewegbar ist. Die Achsen spannen dabei einen dreidimensionalen Raum auf.

Der Patiententisch 15 ist in Ausführungen der Erfindung außerdem wenigstens um die Achse in y-Richtung drehbar gelagert.

Der Patientenaufnahmebereich 12 umfasst eine den Patientenaufnahmebereich 12 umgebenden Umhausung 36 mit einer Innenseite bzw. Innenwand 37. Die den Patientenaufnahmebereich 12 umgebenden Umhausung 36 ist im vorliegenden Ausführungsbeispiel einteilig mit der Hochfrequenzantenneneinheit 20, insbesondere einer dem Patientenaufnahmebereich 12 zugewandten Seite der Hochfrequenzantenneneinheit 20, ausgebildet. In einer alternativen Ausgestaltung der Erfindung kann die den Patientenaufnahmebereich 12 umgebende Umhausung 36 auch ein von der Hochfrequenzantenneneinheit 20 separaten Einheit bilden.

Die Magneteinheit 11, umfasst einen supraleitenden Grundmagneten 16 zum Erzeugen eines starken und insbesondere konstanten Grundmagnetfelds 17. Weiterhin weist die Magneteinheit 11, eine Gradientenspuleneinheit 18 zum Erzeugen von Magnetfeldgradienten auf, die für eine Ortskodierung während einer Bildgebung verwendet werden. Die Gradientenspuleneinheit 18 wird mittels einer Gradientensteuereinheit 19 des Magnetresonanztomographen 10 gesteuert. Die Magneteinheit 11, umfasst weiterhin eine Hochfrequenzantenneneinheit 20 zu einer Anregung einer Polarisation, die sich in dem von dem Grundmagneten 16 erzeugten Grundmagnetfeld 17 einstellt. Die Hochfrequenzantenneneinheit 20 wird von einer Hochfrequenzantennensteuereinheit 21 des Magnetresonanztomographen 10 gesteuert und strahlt hochfrequente Magnetresonanzsequenzen in den Patientenaufnahmebereich 12 des Magnetresonanztomographen 10 ein.

Zu einer Steuerung des Grundmagneten 16, der Gradientensteuereinheit 19 und zur Steuerung der Hochfrequenzantennensteuereinheit 21 weist der Magnetresonanztomograph 10 eine Systemsteuereinheit 22 auf. Die Systemsteuereinheit 22 wird von einer Recheneinheit des Magnetresonanztomographen 10 umfasst. Die Systemsteuereinheit 22 steuert zentral das den Magnetresonanztomographen 10, wie beispielsweise das Durchführen einer vorbestimmten bildgebenden Gradientenechosequenz. Zudem umfasst die Systemsteuereinheit 22 eine nicht näher dargestellte Auswerteeinheit zu einer Auswertung von medizinischen Bilddaten bzw. Magnet-Resonanz-Tomographie (Akronym: MRT) Bilddaten, die während der Magnetresonanzuntersuchung bzw. Untersuchung erfasst werden.

Des Weiteren umfasst der Magnetresonanztomograph 10 eine Benutzerschnittstelle 23, die mit der Systemsteuereinheit 22 verbunden ist. Steuerinformationen wie beispielsweise Bildgebungsparameter, sowie rekonstruierte MRT-Bilddaten können auf einer Anzeigeeinheit 24, beispielsweise auf zumindest einem Monitor, der Benutzerschnittstelle 23 für ein medizinisches Bedienpersonal bzw. medizinisches Personal angezeigt werden. Weiterhin weist die Benutzerschnittstelle 23 eine Eingabeeinheit 25 auf, mittels der Informationen und/oder Parameter während eines Messvorgangs von dem medizinischen Bedienpersonal eingegeben werden können.

Die Magneteinheit 11 der Magnetresonanzvorrichtung 10 ist zusammen mit der Patientenlagerungsvorrichtung 14 innerhalb eines MRT-Raums 26 angeordnet. Die Systemsteuereinheit 22 dagegen ist zusammen mit der Benutzerschnittstelle 23 innerhalb eines Kontrollraums 27 angeordnet. Der Kontrollraum 27 ist getrennt von dem MRT-Raum 26 ausgebildet. Insbesondere ist der MRT-Raum 26 hinsichtlich einer Hochfrequenzstrahlung von dem Kontrollraum 27 abgeschirmt. Während einer Untersuchung befindet sich der Patient 13 innerhalb des MRT-Raums 26, dagegen befindet sich das medizinische Bedienpersonal zumeist innerhalb des Kontrollraums 27. Der MRT-Raum 26 und der Kontrollraum 27 in Kombination bilden den MRT-Untersuchungsraum.

Für eine Kommunikation und/oder einen Informationsaustausch des Patienten 13 mit dem medizinischen Bedienpersonal während einer Untersuchung weist der Magnetresonanztomograph 10 eine Kommunikationseinheit 28 auf. Die Kommunikationseinheit 28 weist auf Bedienerseite eine als Bedienerkonsole 29 ausgebildetes Kommunikationselement auf. Das Kommunikationselement, insbesondere die Bedienerkonsole 29, ist bevorzugt innerhalb des Kontrollraums 27 angeordnet. Die Bedienerkonsole 29 weist ein Eingabeelement 30 und ein Ausgabeelement 31 auf. Das Eingabeelement 30 und/oder das Ausgabeelement 31 kann dabei als akustisches und/oder visuelles Eingabeelement 30 und/oder Ausgabeelement 31 ausgebildet sein.

Des Weiteren weist die Kommunikationseinheit 28 auf Patientenseite ein erstes Kommunikationselement auf, das als Eingabeelement 32 ausgebildet ist. Mittels des Eingabeelements 32 kann der Patient 13 dem Bediener, insbesondere dem medizinischen Bedienpersonal, ein Befinden, wie beispielsweise ein Unwohlsein, während der Untersuchung mitteilen. Im vorliegenden Ausführungsbeispiel ist das Eingabeelement 32 als Patientenrufball bzw. Alarmball ausgebildet. Grundsätzlich sind jedoch weitere, dem Fachmann als sinnvoll erscheinende Eingabeelemente 32, wie beispielsweise ein Mikrofon usw. in einer weiteren Ausbildung der Kommunikationseinheit 28 möglich.

Der Magnetresonanztomograph 10 umfasst außerdem ein Schienensystem 43, welches zum Führen einer Vorschubeinheit 42 ausgebildet ist. Die Vorschubeinheit 42 ist wiederum zum Halten eines medizintechnischen Instrumentes 41 ausgebildet. Dafür wird die Vorschubeinheit 42 mit dem medizintechnischen Instrument 41 verbunden. Mit anderen Worten wird das medizintechnische Instrument 41 an der Vorschubeinheit 42 befestigt. Die Vorschubeinheit 42 kann entlang des Schienensystems 43 verfahren werden. Das Schienensystem 43 verläuft insbesondere entlang eines Kreisbogens auf der dem Patienten zugewandten Innenseite der Umhausung 36. Der Kreisbogen kann beispielsweise einen Winkel von 10°, 20°, 30°, 40°, 50°, 60°, 70°, 80°, 90°, 120°, 180°, 210°, 240° oder 270° abdecken. Insbesondere kann das Schienensystem 43 ringförmig ausgebildet sein, also einen Kreisbogen von 360° abdecken.

Die Vorschubeinheit 42 kann insbesondere mittels Rollen entlang des Schienensystems 43 bewegbar sein. Alternativ kann die Vorschubeinheit 42 mittel anderer Lager entlang des Schienensystems 43 bewegbar sein.

In dem dargestellten Ausführungsbeispiel ist das Schienensystem 43 in einem innenumfänglichen Spalt 44 der Magneteinheit 11 angeordnet. Insbesondere ist das Schienensystem 43 in einem innenumfänglichen Spalt 44 angeordnet, der durch die Gradientenspuleneinheit 18 und die Hochfrequenzantenneneinheit 20 gebildet wird. Dafür ist die Gradientenspuleneinheit 18 und die Hochfrequenzantenneneinheit 20 in zwei Teilstücken unterteilt, die durch den innenumfänglichen Spalt 44 voneinander beabstandet sind. Alternativ kann das Schienensystem 43 kann entlang der Innenwand 37 der Umhausung 36 des Patientenaufnahmebereiches 12 angeordnet sein.

Das medizintechnische Instrument 41 ist dabei zum Durchführen einer Intervention ausgebildet. Das medizintechnische Instrument 41 ist dabei insbesondere eine medizinische Nadel, insbesondere eine Biopsienadel. Dann ist die Intervention insbesondere eine Biopsie. Alternativ kann das medizintechnische Instrument 41 ein Katheter und/oder eine hochintensive, fokussierte Ultraschallsonde sein.

Die Vorschubeinheit 42 ist in Ausführungen der Erfindung dazu ausgebildet, das medizintechnische Instrument 41 radial, senkrecht zu dem Schienensystem 43 zu verstellen. Mit anderen Worten kann die Vorschubeinheit 42 das medizintechnische Instrument 41 in Richtung des Patienten 13 bewegen. Insbesondere kann dafür die Vorschubeinheit 42 das medizintechnische Instrument 41 mit einem Piezomotor bewegen.

Die Vorschubeinheit 42 ist in Ausführungen der Erfindung dazu ausgebildet das medizintechnische Instrument 41, um eine Achse senkrecht zu dem Schienensystem 43 zu drehen und/oder relativ zu dieser Achse zu verschwenken. Auf diese Weise kann eine Ausrichtung des medizintechnischen Instrumentes 41 relativ zu dem Patienten 13 angepasst werden. Insbesondere kann die Vorschubeinheit 42 das medizintechnische Instrument 41 mittels einem Piezomotor drehen und/oder schwenken.

Die Vorschubeinheit 42 kann insbesondere ein Auslösesystem umfassen. Das Auslösesystem umfasst einen Kraftsensor, der eine auf das medizintechnische Instrument 41 wirkende Kraft misst bzw. erfasst. Insbesondere misst der Kraftsensor eine senkrecht zu der Trajektorie auf das medizintechnische Instrument 41 wirkende Kraft. Das Auslösesystem löst das medizintechnische Instrument 41 von der Vorschubeinheit 42, wenn diese Kraft einen Grenzwert überschreitet.

Der Magnetresonanztomograph 10 ist dazu ausgebildet, mittels einer Bewegung des Patiententisches 15 entlang der oben beschriebenen Achsen und/oder einer Rotation um wenigstens eine der oben beschriebenen Achsen und einer Bewegung des medizintechnischen Instrumentes 41 entlang des Schienensystems 43 mittels der Vorschubeinheit 42, das medizintechnische Instrument 41 entlang einer vorgegebenen Trajektorie in dem Patienten 13 zu führen bzw. zu bewegen.

Der dargestellte Magnetresonanztomograph 10 kann selbstverständlich weitere Komponenten umfassen, die Magnetresonanztomographen 10 gewöhnlich aufweisen. Eine allgemeine Funktionsweise eines MRT-Systems 1 ist zudem dem Fachmann bekannt, so dass auf eine detaillierte Beschreibung der weiteren Komponenten verzichtet wird.

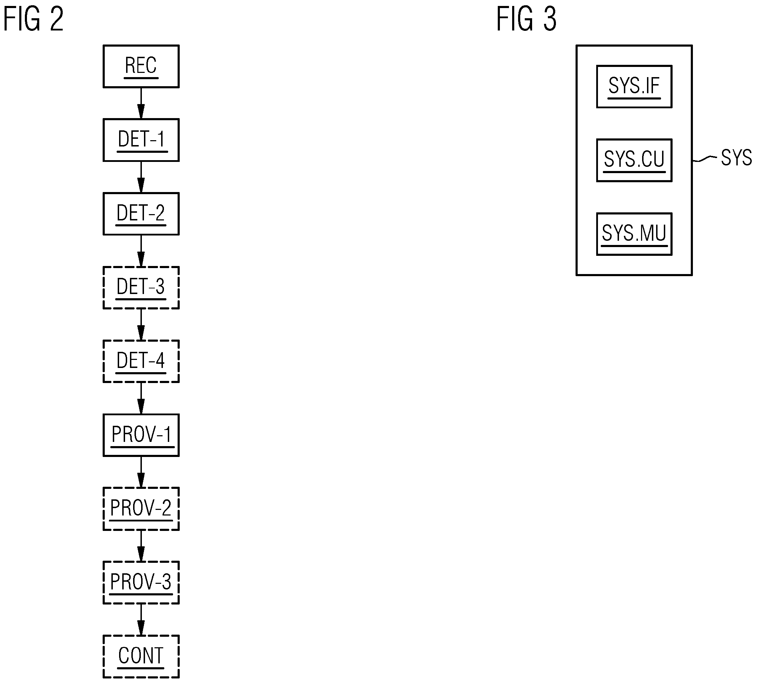

**Figur 2** zeigt ein Ausführungsbeispiel eines computerimplementierten Verfahrens zum Planen einer Bewegung eines medizintechnischen Instrumentes 41 in einem Patientenaufnahmebereich 12 eines Magnetresonanztomographen 10 entlang einer Trajektorie.

Der Magnetresonanztomograph 10 ist dabei wie bezüglich Figur 1 beschrieben ausgebildet.

In einem Verfahrensschritt eines Empfangens REC der Trajektorie wird eine Trajektorie mittels einer Schnittstelle SYS.IF empfangen. Die Trajektorie gibt dabei relativ zu einem Patienten 13 vor, wie das medizintechnische Instrumente 41 geführt werden soll. Mit anderen Worten gibt die Trajektorie einen Weg innerhalb des Patienten 13 für das medizintechnische Instrument 41 vor. Mit anderen Worten gibt die Trajektorie einen Weg des medizintechnischen Instrumentes 41 von einer Einstichstelle in den Patienten 13 bis zu einem Zielort vor. Der Weg kann dabei geradlinig oder gekrümmt sein. Alternativ kann der Weg entlang eines Blutgefäßes verlaufen. Der Zielort ist dabei der Ort in dem Patienten 13, an welchem eine Intervention mit Hilfe des medizintechnischen Instrumentes 41 durchgeführt werden soll. Das medizintechnische Instrument 41 ist dabei wie bezüglich Figur 1 beschrieben ausgeführt.

In einem Verfahrensschritt eines Bestimmens DET-1 von wenigstens einem Tischparameters einer Tischbewegung des Patiententisches 15 des Magnetresonanztomographen 10 wird der wenigstens eine Tischparameter mittels einer Recheneinheit SYS.CU bestimmt. Der Patiententisch 15 ist dabei wie bezüglich Figur 1 beschrieben ausgebildet. Insbesondere ist der Patiententisch 15 wenigstens entlang von drei Achsen bewegbar ausgebildet. Insbesondere kann der Patiententisch 15 um wenigstens eine der Achsen außerdem rotierbar ausgebildet sein. Der wenigstens eine Tischparameter beschreibt dabei eine Bewegung des Patiententisches 15 entlang dieser Achsen und/oder eine Rotation des Patiententisches 15 um diese Achsen. Der wenigstens eine Tischparameter wird dabei basierend auf der Trajektorie bestimmt.

In einem Verfahrensschritt eines Bestimmens DET-2 von wenigstens einem Schienenparameter eines Bewegung des medizintechnischen Instrumentes 41 entlang des Schienensystems 43 wird der wenigstens eine Schienenparameter mittels der Recheneinheit SYS.CU bestimmt. Das Schienensystem 43 ist dabei wie bezüglich Figur 1 beschrieben ausgebildet. Das medizintechnische Instrument 41 ist dabei wie bezüglich Figur 1 beschrieben an der Vorschubeinheit 42 befestigt. Die Vorschubeinheit 42 kann dabei entlang des Schienensystems 43 verstellt bzw. bewegt bzw. geführt werden. Durch das Bewegen bzw. Verstellen bzw. Führen der Vorschubeinheit 42 wird auch das medizintechnische Instrument 41 entlang des Schienensystems 43 verstellt bzw. bewegt bzw. geführt. Der wenigstens eine Schienenparameter beschreibt dabei die Bewegung des medizintechnischen Instrumentes 41 entlang des Schienensystems 43. Insbesondere kann der wenigstens eine Schienenparameter wenigstens eine Position des medizintechnischen Instrumentes 41 entlang des Schienensystems vorgeben. Der wenigstens eine Schienenparameter wird dabei basierend auf der Trajektorie bestimmt.

Der wenigstens eine Tischparameter und der wenigstens eine Schienenparameter sind dabei derart ausgebildet, dass das medizintechnische Instrument 41 in dem auf dem Patiententisch 15 gelagerten Patienten 13 entlang der Trajektorie mittels der aus den Parametern resultierenden Tischbewegung und der Bewegung des medizintechnischen Instrumentes 41 entlang des Schienensystems 43 bewegt bzw. geführt werden kann.

In einem weiteren Verfahrensschritt eines Bereitstellens PROV-1 des wenigstens einen Tischparameters und des wenigstens einen Schienenparameters werden die beiden Parameter mittels einer Schnittstelle SYS.IF bereitgestellt. Insbesondere können die beiden Parameter dem Magnetresonanztomographen 10 bereitgestellt werden. Insbesondere können die beiden Parameter derart bereitgestellt werden, dass der Magnetresonanztomograph 10 die Tischbewegung und die Bewegung des medizintechnischen Instrumentes 41 entlang des Schienensystems 43 basierend auf dem wenigstens einen Tischparameter und dem wenigstens einen Schienenparameter ausführen kann.

Der wenigstens eine Tischparameter und der wenigstens eine Schienenparameter werden insbesondere im Vorfeld einer Intervention bestimmt und bereitgestellt.

In einem optionalen Verfahrensschritt eines Bestimmens DET-3 wenigstens eines Atemparameters der Tischbewegung wird mittels der Recheneinheit SYS.CU der wenigstens eine Atemparameter bestimmt. Der wenigstens eine Atemparameter gibt dabei eine Tischbewegung vor, die der Tischbewegung basierend auf dem wenigstens einen Tischparameter überlagert wird. Die Tischbewegung basierend auf dem wenigstens einen Atemparameter ist derart ausgebildet, dass eine Atembewegung des Patienten 13 mittels der auf dem wenigstens einen Atemparameter basierend Tischbewegung kompensiert wird. Mit anderen Worten ist die auf dem wenigstens einen Atemparameter basierende Tischbewegung komplementär zu der Bewegung des Patienten 13, welche durch seine Atmung bzw. der Atembewegung verursacht wird, ausgebildet.

Der wenigstens eine Atemparameter kann insbesondere während einer Durchführung der Intervention bestimmt werden. Insbesondere kann der wenigstens eine Atemparameter basierend auf Sensordaten bestimmt werden. Die Sensordaten können dabei mittels wenigstens eines Sensors, der auf dem Patienten 13 befestigt ist, erfasst werden. Alternativ kann der wenigstens eine Atemparameter im Vorfeld der Intervention bestimmt werden. Insbesondere kann der wenigstens eine Atemparameter dabei basierend auf Abschätzungen der Atembewegungen des Patienten bestimmt werden. Insbesondere kann er wenigstens eine Atemparameter dabei auf einer periodischen Abschätzung basieren. Alternativ kann der wenigstens eine Atemparameter mittels einer trainierten Funktion bestimmt werden.

In einem weiteren optionalen Verfahrensschritt eines Bereitstellens PROV-2 wird der wenigstens eine Atemparameter mittels der Schnittstelle SYS.IF bereitgestellt. Insbesondere wird dabei der wenigstens eine Atemparameter dem Magnetresonanztomographen 10 derart bereitgestellt, dass eine Bewegung des Patiententisches 15 basierend auf dem wenigstens einen Atemparameter ausgeführt werden kann. Alternativ kann der wenigstens eine Atemparameter einer Datenbank bereitgestellt werden. Insbesondere kann dann der Atemparameter von dem Magnetresonanztomographen 10 von der Datenbank abgerufen werden.

In einem weiteren optionalen Verfahrensschritt eines Bestimmens DET-4 wenigstens eines Vorschubparameters wird der wenigstens eine Vorschubparameter mittels der Recheneinheit SYS.CU bestimmt. Der wenigstens eine Vorschubparameter wird dabei basierend auf der Trajektorie bestimmt. Dabei ist die Vorschubeinheit 42 dazu ausgebildet, mittels des wenigstens einen Vorschubparameters das medizintechnische Instrument 41 in Kombination mit der auf dem wenigstens einen Tischparameter basierenden Tischbewegung des Patiententisches 15 und der auf dem wenigstens einen Schienenparameter basierenden Bewegung des medizintechnischen Instrumentes 41 entlang des Schienensystems 43 entlang der Trajektorie zu führen.

Insbesondere kann der wenigstens eine Vorschubparameter, eine Bewegung des medizintechnischen Instrumentes 41 senkrecht zu dem Schienensystem 43 definieren. Die Bewegung senkrecht zu dem Schienensystem 43 kann dabei insbesondere mittels eines Piezo-Motors ausgeführt werden. Insbesondere umfasst dann die Vorschubeinheit 42 den Piezo-Motor.

In Ausführungen der Erfindung kann der wenigstens eine Vorschubparameter alternativ oder zusätzlich eine Drehung und/oder ein Schwenken des medizintechnischen Instrumentes 41 um bzw. bezüglich einer Achse senkrecht zu dem Schienensystem 43 mittels der Vorschubeinheit 42 definieren. Insbesondere kann zum Ausführen der Drehung und/oder des Schwenkens die Vorschubeinheit 42 einen Piezo-Motor umfassen.

Der wenigstens eine Vorschubparameter kann dabei im Vorfeld der Intervention bestimmt werden.

In einem weiteren optionalen Verfahrensschritt eines Bereitstellens PROV-3 des wenigstens einen Vorschubparameters wird der wenigstens eine Vorschubparameter mittels der Schnittstelle SYS.IF bereitgestellt. Insbesondere wird der wenigstens eine Vorschubparameter derart bereitgestellt, dass basierend auf dem wenigstens einen Vorschubparameter, dem wenigstens einen Tischparameter und dem wenigstens einen Schienenparameter das medizintechnische Instrument 41 entlang der Trajektorie in dem Patienten 13 bewegt werden kann.

In einem weiteren optionalen Verfahrensschritt eines Steuerns CONT des Magnetresonanztomographen 10 basierend auf dem wenigstens einen Tischparameter und dem wenigstens einen Schienenparameter und optionale zusätzlich basierend auf dem wenigstens einen Vorschubparameter und/oder dem wenigstens einen Atemparameter wird der Magnetresonanztomograph 10 basierend auf den bereitgestellten Parametern gesteuert. Insbesondere wird dabei die Bewegung des Patiententisches 15 bzw. die Tischbewegung und die Bewegung des medizintechnischen Instrumentes 41 entlang des Schienensystems 43 und optional eine Bewegung des medizintechnischen Instrumentes 41 mittels der Vorschubeinheit 42 gesteuert. Die Bewegungen werden dabei derart gesteuert, dass das medizintechnische Instrument 41, wenn ein Patient 13 auf dem Patiententisch 15 positioniert ist, entlang der Trajektorie in dem Patienten 13 bewegt wird.

**Figur 3** zeigt ein Planungssystem SYS zum Planen einer Bewegung eines medizintechnischen Instrumentes 41 in einem Patientenaufnahmebereich 12 eines Magnetresonanztomographen 10 entlang einer Trajektorie.

Das dargestellte Planungssystem SYS zum Planen einer Bewegung eines medizintechnischen Instrumentes 41 in einem Patientenaufnahmebereich 12 eines Magnetresonanztomographen 10 entlang einer Trajektorie ist dazu ausgebildet ein erfindungsgemäßes Verfahren zum Planen einer Bewegung eines medizintechnischen Instrumentes 41 in einem Patientenaufnahmebereich 12 eines Magnetresonanztomographen 10 entlang einer Trajektorie auszuführen. Das Planungssystem SYS umfasst eine Schnittstelle SYS.IF, eine Recheneinheit SYS.CU und eine Speichereinheit SYS.MU.

Das Planungssystem SYS kann insbesondere ein Computer, ein Mikrocontroller oder ein integrierter Schaltkreis (integrated circuit, IC) sein. Alternativ kann das Planungssystem SYS ein reales oder virtuelles Computer-Netzwerk sein (eine technische Bezeichnung für ein reales Computer-Netzwerk ist "Cluster", eine technische Bezeichnung für ein virtuelles Computer-Netzwerk ist "Cloud"). Das Planungssystem SYS kann als virtuelles System ausgebildet sein, welches auf einem Computer oder einem realen Computer-Netzwerk oder einem virtuellen Computer-Netzwerk ausgeführt wird (eine technische Bezeichnung ist "Virtualization").

Die Schnittstelle SYS.IF kann eine Hardware- oder Software-Schnittstelle sein (beispielsweise ein PCI bus, USB oder Firewire). Die Recheneinheit SYS.CU kann Hardware und/oder Software Bestandteile umfassen, beispielsweise einen Mikroprozessor oder einen sogenannten FPGA (Field Programmable Gate Way). Die Speichereinheit SYS.MU kann als nicht permanent arbeitender Arbeitsspeicher (Random Access Memory, RAM) oder als permanenter Massenspeicher (Festplatte, USB-Stick, SD-Karte, Solid State Disk (SSD)) ausgebildet sein.

Die Schnittstelle SYS.IF kann insbesondere eine Mehrzahl an Sub-Schnittstellen umfassen, die unterschiedliche Verfahrensschritte des jeweiligen erfindungsgemäßen Verfahrens ausführen. Mit anderen Worten kann die Schnittstelle SYS.IF als eine Mehrzahl an Schnittstellen SYS.IF ausgebildet sein. Die Recheneinheit SYS.CU kann insbesondere eine Mehrzahl an Sub-Recheneinheiten umfassen, die unterschiedliche Verfahrensschritte des jeweiligen erfindungsgemäßen Verfahrens ausführen. Mit anderen Worten kann die Recheneinheit SYS.CU als eine Mehrzahl an Recheneinheiten SYS.CU ausgebildet sein.

Wo noch nicht explizit geschehen, jedoch sinnvoll und im Sinne der Erfindung, können einzelne Ausführungsbeispiele, einzelne ihrer Teilaspekte oder Merkmale miteinander kombiniert bzw. ausgetauscht werden, ohne den Rahmen der hiesigen Erfindung zu verlassen. Mit Bezug zu einem Ausführungsbeispiel beschriebene Vorteile der Erfindung treffen ohne explizite Nennung, wo übertragbar, auch auf andere Ausführungsbeispiele zu.

## Patentansprüche

1. Magnetresonanztomograph (10) umfassend:
- einen Patiententisch (15),
- einen Patientenaufnahmebereich (12),
- eine Vorschubeinheit (42), die dazu ausgebildet ist, ein medizintechnisches Instrument (41) zu führen,
- ein Schienensystem (43) zum Führen der Vorschubeinheit (42),
wobei das Schienensystem (43) innenumfänglich entlang einer Innenseite (37) des Patientenaufnahmebereiches (12) verläuft, wobei der Patiententisch (15) dazu ausgebildet ist, einen Patienten (13) in dem Patientenaufnahmebereich (12) zu positionieren,
wobei der Patiententisch (15) derart bewegbar ausgebildet ist, dass das medizintechnische Instrument (41) anhand einer Bewegung des Patiententisches (15) in dem Patienten (13) entlang einer Trajektorie geführt werden kann.

2. Magnetresonanztomograph (10) nach Anspruch 1,
wobei der Patiententisch (15) wenigstens drei Achsen umfasst, wobei die Achsen derart ausgebildet sind, dass das medizintechnische Instrument (41) durch eine Bewegung des Patiententisches (15) entlang der Trajektorie bewegbar ist,
wobei die Bewegung des Patiententisches (15) durch eine Linearkombination von Bewegungen entlang der Achsen und/oder einer Rotation um wenigstens eine der Achsen erzeugt wird.

3. Magnetresonanztomograph (10) nach einem der vorhergehenden Ansprüche,
wobei die drei Achsen derart ausgerichtet sind, dass sie einen dreidimensionalen Raum aufspannen.

4. Magnetresonanztomograph (10) nach einem der vorhergehenden Ansprüche,
wobei das medizintechnische Instrument (41) eine medizinische Nadel, insbesondere eine Biopsienadel ist.

5. Magnetresonanztomograph (10) nach einem der vorhergehenden Ansprüche,
wobei das Schienensystem (43) entlang eines Kreisbogens verläuft.

6. Magnetresonanztomograph (10) nach einem der vorhergehenden Ansprüche,
wobei das Schienensystem (43) ringförmig ausgebildet ist.

7. Magnetresonanztomograph (10) nach einem der vorhergehenden Ansprüche,
wobei der Magnetresonanztomograph (10) außerdem eine Magneteinheit (11) umfasst,
wobei der Patientenaufnahmebereich (12) von der Magneteinheit (11) umschlossen wird,
wobei die Magneteinheit (11) einen innenumfänglichen Spalt (44) bildet,
wobei das Schienensystem (43) in dem Spalt (44) angeordnet ist.

8. Magnetresonanztomograph (10) nach einem der vorhergehenden Ansprüche,
wobei die Vorschubeinheit (42) dazu ausgebildet ist, das medizintechnische Instrument (41) senkrecht zu dem Schienensystem (43) zu bewegen.

9. Magnetresonanztomograph (10) nach einem der vorhergehenden Ansprüche,
wobei die Vorschubeinheit (42) dazu ausgebildet ist, das medizintechnische Instrument (41) zu drehen und/oder zu verschwenken.

10. Magnetresonanztomograph (10) nach einem der Ansprüche 8 oder 9,
wobei die Vorschubeinheit (42) wenigstens einen Piezomotor umfasst, welcher dazu ausgebildet ist, das medizintechnische Instrument (41) zu bewegen.

11. Magnetresonanztomograph (10) nach einem der vorhergehenden Ansprüche,
wobei die Vorschubeinheit (42) ein Auslösesystem umfasst,
wobei das Auslösesystem ausgebildet ist, das medizintechnische Instrument (41) von der Vorschubeinheit (42) zu lösen, wenn eine auf das medizintechnische Instrument (41) wirkende Kraft einen Grenzwert überschreitet.

12. Computerimplementiertes Verfahren zum Planen einer Bewegung eines medizintechnischen Instrumentes (41) in einem Patientenaufnahmebereich (12) eines Magnetresonanztomographen (10) gemäß einem der Ansprüche 1 bis 10 entlang einer Trajektorie,
umfassend folgende Verfahrensschritte:
- Empfangen (REC) einer Trajektorie,
wobei die Trajektorie relativ zu einem Patienten (13) vorgibt, wie das medizintechnische Instrument (41) geführt werden soll,
- Bestimmen (DET-1) von wenigstens einem Tischparameter einer Tischbewegung des Patiententisches (15) in Abhängigkeit der Trajektorie,
- Bestimmen (DET-2) von wenigstens einem Schienenparameter einer Bewegung des medizintechnischen Instrumentes (41) entlang des Schienensystems (43) in Abhängigkeit der Trajektorie,
wobei der wenigstens eine Tischparameter und der wenigstens eine Schienenparameter derart ausgebildet sind, dass das medizintechnische Instrument (41) in einem auf dem Patiententisch (15) gelagerten Patienten (13) entlang der Trajektorie mittels der Tischbewegung und der Bewegung des medizintechnischen Instrumentes (41) entlang des Schienensystems (43) geführt werden kann,
- Bereitstellen (PROV-1) des wenigstens einen Tischparameters der Tischbewegung und des wenigstens einen Schienenparameters der Bewegung des medizintechnischen Instrumentes (41) entlang des Schienensystems (43).

13. Verfahren nach Anspruch 12,
außerdem umfassend folgende Verfahrensschritte:
- Bestimmen (DET-3) wenigstens eines Atemparameters der Tischbewegung,
wobei der Atemparameter dazu ausgebildet ist, den Patiententisch (15) zur Kompensation einer Atembewegung des Patienten (13) zu bewegen,
- Bereitstellen (PROV-2) des wenigstens einen Atemparameters der Tischbewegung.

14. Verfahren nach einem der Ansprüche 12 oder 13,
außerdem umfassend folgende Verfahrensschritte:
- Bestimmen (DET-4) wenigstens eines Vorschubparameters in Abhängigkeit der Trajektorie,
wobei die Vorschubeinheit (42) dazu ausgebildet ist, mittels des wenigstens einen Vorschubparameters das medizintechnische Instrument (41) in Kombination mit der Tischbewegung des Patiententisches (15) und der Bewegung des medizintechnischen Instrumentes (41) entlang des Schienensystems (43) entlang der Trajektorie zu führen,
- Bereitstellen (PROV-3) des wenigstens einen Vorschubparameters.

15. Planungssystem (SYS) zum Planen einer Bewegung eines medizintechnischen Instrumentes (41) in einem Patientenaufnahmebereich (12) eines Magnetresonanztomographen (10) gemäß einem der Ansprüche 1 bis 11 entlang einer Trajektorie,
wobei das Planungssystem (SYS) eine Schnittstelle (SYS.IF) und eine Recheneinheit (SYS.CU) umfasst,
wobei die Schnittstelle (SYS.IF) und die Recheneinheit (SYS.CU) zum Ausführen der folgenden Verfahrensschritte ausgebildet sind:
- Empfangen (REC) einer Trajektorie,
wobei die Trajektorie relativ zu einem Patienten (13) vorgibt, wie das medizintechnische Instrument (41) geführt werden soll,
- Bestimmen (DET-1) von wenigstens einem Tischparameter einer Tischbewegung des Patiententisches (15),
- Bestimmen (DET-2) von wenigstens einem Schienenparameter einer Bewegung des medizintechnischen Instrumentes (41) entlang des Schienensystems (43),
wobei der wenigstens eine Tischparameter und der wenigstens eine Schienenparameter derart ausgebildet sind, dass das medizintechnische Instrument (41) in einem auf dem Patiententisch (15) gelagerten Patienten (13) entlang der Trajektorie mittels der Tischbewegung und der Bewegung des medizintechnischen Instrumentes (41) entlang des Schienensystems (43) geführt werden kann,
- Bereitstellen (PROV-1) des wenigstens einen Tischparameters der Tischbewegung und des wenigstens einen Schienenparameters der Bewegung des medizintechnischen Instrumentes (41) entlang des Schienensystems (43).

16. Computerprogrammprodukt mit einem Computerprogramm, welches direkt in einen Speicher (SYS.MU) eines Planungssystems (SYS) ladbar ist, mit Programmabschnitten, um alle Schritte des Verfahrens nach einem der Ansprüche 12 bis 14 auszuführen, wenn die Programmabschnitte von dem Planungssystem (SYS) ausgeführt werden

17. Computerlesbares Speichermedium, auf welchem von einem Planungssystem (SYS) lesbare und ausführbare Programmabschnitte gespeichert sind, um alle Schritte des Verfahrens nach einem der Ansprüche 12 bis 14 auszuführen, wenn die Programmabschnitte von dem Planungssystem (SYS) ausgeführt werden.
